Europäisches Patentamt

European Patent Office

Office européen des brevets

(11) Publication number: **0 323 562 B1**

(12) **EUROPEAN PATENT SPECIFICATION**

(45) Date of publication of patent specification: **08.07.92**

(51) Int. Cl.5: **C12Q 1/02**, C12Q 1/54, G01N 33/48, C12M 1/40

(21) Application number: **88119448.4**

(22) Date of filing: **23.11.88**

(54) **A method for monitoring a microbiological process.**

(30) Priority: **17.12.87 SE 8705046**

(43) Date of publication of application:
**12.07.89 Bulletin 89/28**

(45) Publication of the grant of the patent:
**08.07.92 Bulletin 92/28**

(84) Designated Contracting States:
**BE CH DE ES FR GB IT LI NL**

(56) References cited:
**EP-A- 0 202 543**
**EP-A- 0 251 475**
**US-A- 4 240 438**
**US-A- 4 266 021**
**US-A- 4 311 789**

**PATENT ABSTRACTS OF JAPAN, vol.9, no.239 (P-391)[1962], 25 September 1985**

**PATENT ABSTRACTS OF JAPAN, vol.9, no.203 (P-381)[1926], 21 August 1985**

**CHEMICAL ABSTRACTS, vol.104, 1986, Columbus, OH (US); M.ARAI et al., p.276, no.221218v**

(73) Proprietor: **GAMBRO AB**
**Post Box 10101**
**S-220 10 Lund(SE)**

(72) Inventor: **Hakansson, Hakan**
**Astrakanvägen 6**
**S-223 56 Lund(SE)**
Inventor: **Holst, Olle**
**Ejdervägen 1 V**
**S-222 33 Lund(SE)**
Inventor: **Mattiasson, Bo**
**Trädgardsmästaren 26**
**S-222 48 Lund(SE)**

(74) Representative: **Boberg, Nils Gunnar Erik**
**Gambro AB Patent Department Box 10101**
**S-220 10 Lund(SE)**

CHEMICAL ABSTRACTS, vol.103, 1985, Columbus, OH (US); p.343, no.174643x

B.D. DAVIS et al., "Microbiology", 3rd edition, chapter 67, 1980, Harper & Row, New York, NY (US); pp. 1268-1271

CHEMICAL ABSTRACTS, vol. 101, 1984, Columbus, OH (US); F.M.LAJOLO et al., p. 540, no. 109478s

CHEMICAL ABSTRACTS, vol.84, 1976, Columbus, OH (US); S.A.FELICETTI et al., p.221, no.86164a

CHEMICAL ABSTRACTS, vol.108, no.5, February 1988, Columbus, OH (US); B.H.CHUNG et al., p.502, no.36314t

## Description

### TECHNICAL FIELD

The present invention relates to a method for monitoring a microbiological process in a reactor wherein a substance is consumed or formed, the concentrations of this substance being measured continuously or intermittently as a measure of the progress or state of the process through transferring a liquid sample from the medium, which is the object of the process, to a point of analysis at a distance from the actual location of processing.

Preferably the method in accordance with the invention is intended to be applied in a biotechnical process to the measurement of glucose formed or consumed. It will be obvious, however, for those versed in the art that it can be applied likewise to many other microbiological or enzymatic processes. It can be applied, for example, in connection with enzymatic degradation of starch with the help of amylase or in protein hydrolysis with the help of enzymes of the protease type.

### BACKGROUND ART

Measurement of the type mentioned above may be carried out in the reactor where the process takes place or outside this reactor. The invention relates to the latter case.

The invention may be said to be a further development of analytical equipment and processes which have been used previously in connection with the measurement of glucose. Such equipment and such processes are described in more detail for example in American patents 4 229 542, 4 311 789, 4 123 353 and 4 266 021 and European patents EP 69 246, EP 69 247, EP 102 457 and EP 102 458.

If in a bioreactor a sample is taken and this is transferred to a point of analysis, there is a risk of the process continuing during the transport, and this possibly under partly altered conditions. The measuring result, therefor, may not give a clear picture of the situation at the instance of sampling. One possibility of solving this problem is by rapid "freezing" of the sample and transporting it in frozen condition. Here too, however, the test result may be affected.

### DISCLOSURE OF INVENTION

The above mentioned problem is solved in accordance with the invention in such a manner that the said liquid sample is mixed with an inhibitor (7) near the point of sampling (4), preferably in the reactor itself, so that the process is hindered from progressing also during the transport from the point of sampling (4) to the point of analysis (18).

The analysis is facilitated if the liquid sample for this purpose is brought to a cell-free state through dialysis or other filtration. The analytical equipment as a result will not have to be affected by the larger complex material which may be present in the liquid sample at the start.

The analysis is facilitated further if the liquid sample is made to pass an enzyme reactor with an enzyme which converts the substance measured to a more readily measureable product or which consumes an easily measurable co-substrate, e g oxygen, which is measured on a simple oxygen electrode.

The reliability of the analysis if facilitated if the liquid sample at equal intervals is conducted past the enzyme reactor directly to the point of analysis for zero setting of the analytical equipment used.

The reliability is facilitated further if the liquid sample at equal intervals is replaced by a calibration solution for the calibration of the analystical equipment used.

As the inhibitor mentioned preferably anyone from the group of cyanides, azides and heavy metal ions, such as copper, silver or mercury ions is chosen.

If the method in accordance with the invention is applied to a biotechnical process for the measurement of glucose formed or consumed, potassium cyanide is used appropriately as an inhibitor.

Alternatively to the above mentioned substances a diluting liquid may be chosen as an inhibitor with a pH-level which is such that the actual process is interrupted.

If the method in accordance with the invention is applied to the enzymatic degradation of starch with the help of amylase, a material just called amylase inhibitor may be used as an inhibitor.

If the method in accordance with the invention is used instead in connection with protein hydrolysis with the help of the enzyme protease, a protease inhibitor, e g soybean trypsin inhibitor (STI) or alpha-2-microglobulin, may be used as an inhibitor.

The analytical equipment in certain cases may be affected less, if the membrane or other filter medium or inhibitor is chosen so in relation to one another that a substantial part of the inhibitor is prevented from passing through the membrane. As a result the risk of an alteration of the measuring result in time is reduced.

In order to obtain a clear picture of a microbiological process it is necessary in many cases for the measurements of the said substance to be combined wit a measurement of the total quantity of dry cell mass in the medium which is the object of the process.

It is preferred to choose as an enzyme reactor one where the enzyme is attached to a solid carrier, e g porous glass beads. By this the quantitiy of enzyme used is reduced compared with the quantity of enzyme which is required on using the same in free form.

In order to avoid the mixing of the inhibitor used with the medium which is the object of the process, it is suitable to use as a sampling instrument a double-lumen-catheter, wherein one lumen is used for transporting the inhibitor to a point near the point of sampling, whilst the other lumen is used for transporting the liquid sample together with the inhibitor to the point of analysis.

It is appropriate to use a double-lumen-catheter consisting of two concentric tubes, the inner tube terminating at a short distance from the tip of the catheter inside the outer tube and where the outer tube appropriately is used for the supply of the said inhibitor.

BRIEF DESCRIPTION OF THE DRAWINGS

Fig 1 shows in schematic form a system of analysis which may be used for the realization of the method in accordance with the invention.

Fig 2 shows the front end of a double-lumen-catheter which may be used in connection with the realization of the method in accordance with the invention.

Fig 3 shows the change in the glucose content during a test fermantation of Saccharomyces cerevisiae.

Fig 4 shows the change in the glucose content during a test fermentation of Escherichia coli.

EQUIPMENT

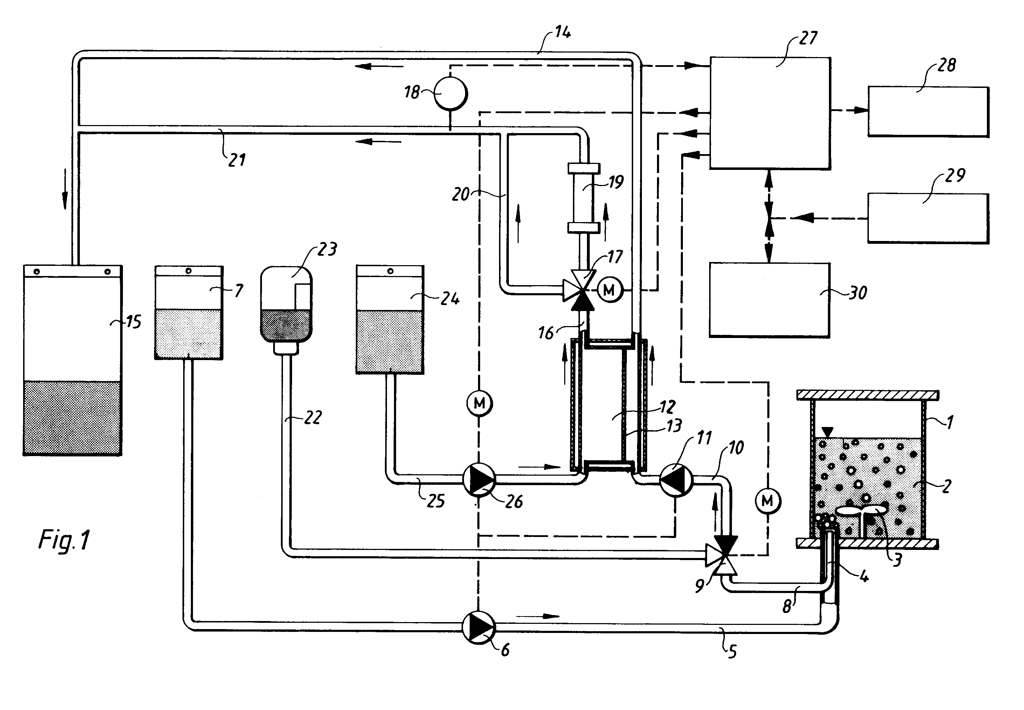

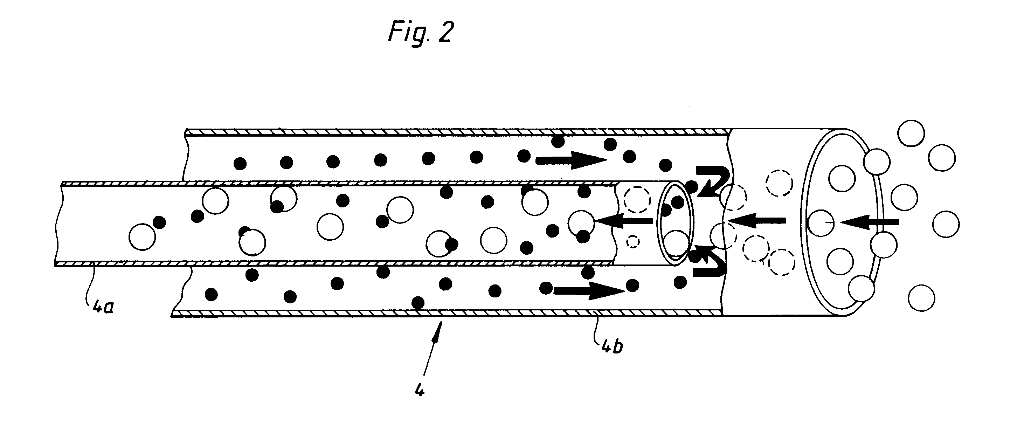

The equipment shown in fig 1 can be used for the monitoring of various biotechnical, e g microbiological or enzymatic processes, but is described for reasons of simplicity in connection with fermentation. In such an application a fermentator designated 1 holds a medium 2 which is stirred by means of a stirrer 3. Samples are taken by means of a double-lumen-catheter 4 whose front end is shown on an enlarged scale in fig 2. An inhibitor is supplied to the catheter from a source 7 via a duct 5 with a pump 6. This inhibitor is mixed with a liquid sample from the fermentor 1 and is passed via a duct 8 to a valve 9. During sampling it is conducted from the valve 9 through a duct 10 with a pump 11 into a dialyser 12 on the one side of a membrane 13 and further through a duct 14 to a waste bag 15. A part of the sample is dialyzed, however, through the membrane 13 and is passed further through a duct 16 to a valve 17. From this valve the sample can be taken either directly to a measuring electrode 18 or via an enzyme reactor 19 to the same measuring electrode. If the liquid sample is led past the enzyme reactor, this is done through the duct 20. From the measuring electrode 18 the liquid sample is conducted through a duct 21 to the waste bag 15.

If the liquid sample is taken past the enzyme reactor 19, the analytical equipment can be set to zero in relation to the quantity of oxygen which is present in the liquid sample ahead of the enzyme reactor, that is to say he quantity of oxygen which does not depend on the glucose content.

When calibration of the system used is required, the valve 9 is changed over so that a calibration solution can be conducted via a duct 22 from a source 23 of such a solution.

The dialysis of the liquid sample takes place against a pure liquid solution which, for example, may consist of sodium chloride dissolved in water. This solution is taken from a source 24 via a duct 25 with a pump 26 to the side of the membrane 13 opposite the sample in the dialyser 12.

The signal measured is passed to a microcomputer 27 which in turn may be adapted to control the pumps and valves forming part of the system. This is indicated by broken lines.

The microcomputer 27, moreover, may be in contact with a display 28 for the reading of the values obtained, a keyboard 29 for the input of desired check values, and possible external computers 30 which, for example, may be adapted to control the fermentation process itself.

In fig 2 is shown the front end of a double-lumen-catheter used in connection with the invention. This catheter consists of an inner tube 4a and an outer tube 4b, the outer tube in the case shown being used for the supply of an inhibitor from a source 7, whereas the inner tube is used to passing this inhibitor together with the sample taken to the dialyser 12. In the design shown the risk of any inhibitor penetrating into the fermentor is minimal. If it is desired to diminish the risk still further, a pressure or flow monitoring of the two ducts 5 and 8 to and from the catheter may be provided, and the analysis be interrupted, if for example the supply to the catheter were to exceed the amount that is withdrawn.

EXAMPLES

Two different microorganisms, one yeast (Saccharomyces cerevisiae) and one bacteria (Escheria coli) have been used in experiments of monitoring in accordance with the invention. This took place in connection with fermentation realized in a fermentor with a working volume of 3 litres (FLC-B-3, Chemoform AB, H}gersten, Sweden).

Saccharomyces cerevisiae was cultivated in a medium containing (g/l): glucose 3.6; $NH_4Cl$ 0.8; $Na_2HPO_4$ 2 $H_2O$ 0.6; $KH_2PO_4$ 0.4; $MgSO_4$ 7 $H_2O$

0.2 and yeast extract 1.0. Magnesium sulphate and glucose were autoclaved separately.

The temperature was kept at 30°C and the pH at 6.0 through automatic titration with the help of 1.0 M NaOH. A few drops of antifoaming agent were added to prevent foaming. Then air flow was kept at approximately 1 vvm (volume per volume and minute) and the stirrer speed was kept at 300 rpm).

The inoculation batch (100 ml) was allowed to grow overnight on a substrate (YMB, Difco, USA) at 30°C in a rotating shaking machine.

Escherichia coli was cultivated at 37°C in a medium containing (g/l): glucose 4.3; $NH_4Cl$ 0.8; $NaHPO_4$ 2 $H_2O$ 0.6; $KH_2PO_4$ 0.4; $MgSO_4$ 7 $H_2O$ 0.2; yeast extract 1.0 together with trace amounts (mg/l): $FeCl_3$ 6 $H_2O$ 8.35; $CaCl_2$ 2 $H_2O$ 0.33; $ZnSO_4$ 7 $H_2O$ 0.09; $CoCl_2$ 2 $H_2O$ 0.09; $CuSO_4$ 5 $H_2O$ 0.08 and $MnSO_4$ 4 $H_2O$ 0.08. Glucose, $MgSO_4$ and $FeCl_3$ were autoclaved separately.

The pH was kept at 7.0 through automatic feed of 1.0 M NaOH. A few drops of antifoaming agent were added to prevent foaming. The air flow was approxiamately 1 vvm and the stirring rate was set at 500 rpm. The inoculation batch (100 ml) was cultivated overnight on a substrate (NB, Difco, USA) at 37°C in a rotating shaking machine. Deionized water was used.

CHEMICALS

Glucose (AnalaR) was obtained from BDH Chemicals Ltd, England. Yeast extract was purchased from Difco, USA. Anti-foaming agent (Adekanol LG-109) was procured from Asahi Electro Chemical Company, Japan. All other chemicals were of analytical quality.

SEPARATE ANALYSES

The dry weight of the cells was determined by filtration of a known volume of fermentation solution through a predried membrane filter (0.2 $\mu$m, Schleicher & Schuell, FRG). The filter was then dried at 105°C in 75 minutes and weighed.

Control samples for glucose determination were taken from the fermentor through a rubber membrane at its bottom. The samples were sterile-filtered (0.2 $\mu$m, Sartorius) within 30 seconds and frozen immediately. The glucose content was determined using a commercially available enzymatic sampling batch (Glucose, Merckotest, Merck, Darmstadt, FRG).

INSTRUMENTATION

The analytical equipment used was a commercially available glucose monitor (Gambro AB, Lund, Sweden) containing the components shown in fig 1. The analytical parts themselves were constituted of a glucose-oxidase-electrode detector of the Clark type.

SAMPLING EQUIPMENT

The sampling equipment was constituted of the double-lumen-catheter described above and shown in fig 2. The flow in the outer duct was maintained at 3 ml/h whilst the flow in the inner duct was maintained at 6 ml/h. The membrane in the dialyser was constituted of a 17 micro thick flat foil of Cuprofan. On the side of the membrane opposite to the sample passed a salt solution at a flow of 60 ml/h. Small molecules such as glucose were able to pass the membrane whereas larger molecules and other particles were kept back. In this manner a cell-free sample was obtainable.

As an inhibitor potassium cyanide was used in both cases.

RESULT

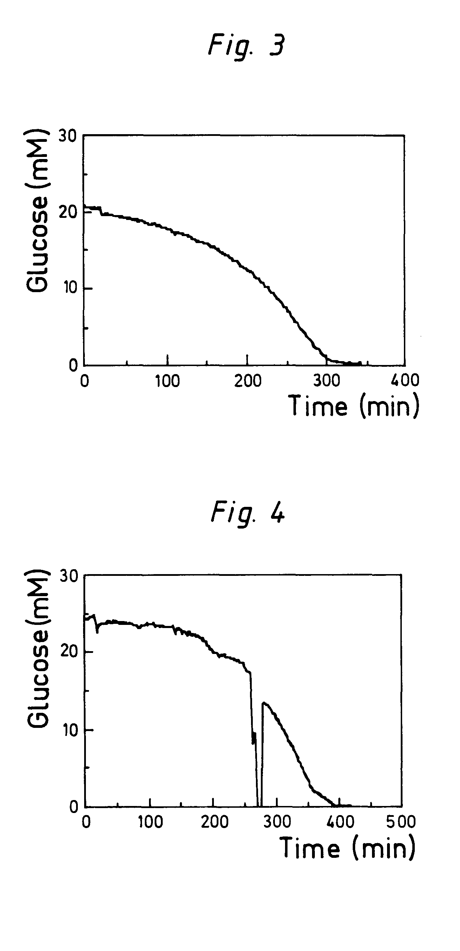

The result is evident from fig 3 and 4, fig 3 relating to the experiment with Saccharomyces cerevisiae and fig 4 to the experiment with Escherichia coli. The drop in signal in fig 4 at approximately 300 minutes is due to recalibration of the system. The values obtained were largely in agreement with the control tests carried out beside them.

SUPPLEMENTARY DESCRIPTION OF EXPERIMENTS

The above mentioned experiments are described in more detail in the attached article "Monitoring of glucose in fermentation processes using a commercial glucose analyser" which will be published in the near future. The article is included therefore in the present description.

Naturally the invention is not limited solely to the embodiments described above, but may be varied within the scope of the following claims. For example, it will be obvious to those versed in the art that it may be applied likewise to many biotechnical processes other than the fermentation process chosen here as an example.

**Claims**

1.  A method for monitoring a microbiological process in a reactor wherein a substance is consumed or formed, the concentration of this substance being measured continuously or intermittently as a measure of the progress or state of the process whereby a liquid sample is transferred from the medium (2) which is the object of the process to a point of analysis (18) at a distance from the actual location of the process, **characterized** in that the said liquid sample is mixed with an inhibitor (7) near the point of sampling (4), preferably in the reactor itself, so as to prevent the process from progressing also during the transport from the point of sampling (4) to the point of analysis (18).

2. A method in accordance with claim 1, **characterized** in that the liquid sample is brought to a cell-free state through dialysis or other filtration.

3. A method in accordance with claim 1 or 2, **characterized** in that the liquid sample is made to pass through an enzyme reactor (19) with an enzyme which converts the substance measured to a more readily measureable product or which consumes an easily measureable co-substrate, e g oxygen, which is measured by means of a simple oxygen electrode.

4. A method in accordance with anyone of the preceding claims, **characterized** in that the liquid sample is carried past the enzyme reactor (19) at equal intervals directly to the point of analysis (18) for zero setting of the analytical equipment used.

5. A method in accordance with anyone of the preceding claims, **characterized** in that the liquid sample is replaced at equal intervals by a calibration solution (23) for the calibration of the analytical equipment used.

6. A method in accordance with anyone of the preceding claims, **characterized** in that as the inhibitor (7) anyone from the group cyanides, azides and heavy metal ions, such as copper, silver or mercury ions is chosen.

7. A method in accordance with claim 6, applied in a biotechnical process for measurement of the glucose formed or consumed, **characterized** in that as an inhibitor potassium cyanide (7) is used.

8. A method in accordance with anyone of claims 1-5, **characterized** in that as the inhibitor (7) a diluting liquid of a pH-level which is such that the actual process is interrupted is chosen.

9. A method in accordance with anyone of claims 1-5, applied to the enzymatic degradation of starch with the help of amylase, **characterized** in that as the inhibitor (7) amylase inhibitor is chosen.

10. A method in accordance with anyone of claims 1-5, applied to protein hydrolysis with the help of enzymes of the protease type, **characterized** in that as the inhibitor (7) a protein inhibitor, e g soybean trypsin inhibitor (STI) or alpha-2-microglobulin is chosen.

11. A method in accordance with claim 2, **characterized** in that the membrane (13) or other filter medium and inhibitor (7) are chosen with respect to one another, such that a substantial part of the inhibitor (7) is prevented from passing through the membrane (13).

12. A method in accordance with anyone of the preceding claims, **characterized** in that the measurements of the said substance are combined with a measurement of the total quantity of dry cell mass in the medium (2) which is the object of the process.

13. A method in accordance with claim 3, **characterized** in that as the enzyme reactor (19) one is chosen where the enzyme is attached to a solid carrier, e g porous glass beads.

14. A method in accordance with any one of the preceding claims, **characterized** in that a double-lumen-catheter (4) is chosen as a sampling instrument, wherein one lumen (4b) is used for transporting the inhibitor to a point near the point of sampling, whereas the other lumen (4a) is used for transporting the liquid sample together with the inhibitor (7) to the point of analysis.

15. A method in accordance with claim 14, **characterized** in that as the said double-lumen-catheter (4) one is chosen which consists of two concentric tubes, the inner tube (4a) closing at a short distance from the tip of the catheter inside the outer tube (4b), and where appropriately the outer tube (4b) is used for the supply of the inhibitor.

**Revendications**

1. Procédé pour le contrôle d'un processus microbiologique dans un réacteur dans lequel une substance est consommée ou formée, la concentration de cette substance étant mesurée de façon continue ou intermittente comme mesure de la progression ou de l'état du processus par lequel on transfère un échantillon liquide du milieu (2) qui fait l'objet du processus vers un point d'analyse (18) à une certaine distance de la localisation effective du processus, caractérisé en ce que l'on mélange ledit échantillon liquide avec un inhibiteur (7) au voisinage du point d'échantillonnage (4), de préférence dans le réacteur lui-même, de manière à empêcher le processus de progresser également pendant le transport à partir du point d'échantillonnage (4) vers le point d'analyse (18).

2. Procédé selon la revendication 1, caractérisé en ce que l'échantillon liquide est amené à un état acellulaire dans une dialyse ou autre filtration.

3. Procédé selon la revendication 1 ou 2, caractérisé en ce que l'on fait passer l'échantillon liquide à travers un réacteur enzymatique (19) avec une enzyme qui transforme la substance mesurée en un produit plus facilement mesurable ou qui consomme un co-substrat facilement mesurable, par exemple l'oxygène, qui est mesuré au moyen d'une électrode à oxygène simple.

4. Procédé selon l'une quelconque des revendications précédentes, caractérisé en ce que l'échantillon liquide est amené au-delà du réacteur enzymatique (19) à des intervalles réguliers directement vers le point d'analyse (18) pour un réglage à zéro de l'équipement analytique utilisé.

5. Procédé selon l'une quelconque des revendications précédentes, caractérisé en ce qu'on remplace l'échantillon liquide à des intervalles réguliers par une solution d'étalonnage (23) pour l'étalonnage de l'équipement analytique utilisé.

6. Procédé selon l'une quelconque des revendications précédentes, caractérisé en ce qu'on choisit comme inhibiteur (7) un corps quelconque choisi dans le groupe des cyanures, azides et ions de métaux lourds, comme les ions cuivre, argent ou mercure.

7. Procédé selon la revendication 6, appliqué à un processus biotechnique pour la mesure du glucose formulé consommé, caractérisé en ce qu'on utilise comme inhibiteur le cyanure de potassium (7).

8. Procédé selon l'une quelconque des revendications 1-5, caractérisé en ce qu'on choisit comme inhibiteur (7) un liquide diluant d'un niveau de pH qui est tel que le processus effectif est interrompu.

9. Procédé selon l'une quelconque des revendications 1-5, appliqué à la dégradation enzymatique de l'amidon à l'aide de l'amylase, caractérisé en ce qu'on choisit comme inhibiteur (7) l'inhibiteur d'amylase.

10. Procédé selon l'une quelconque des revendications 1-5, appliqué à l'hydrolyse des protéines à l'aide d'enzymes du type protéase, caractérisé en ce qu'on choisit comme inhibiteur (7) un inhibiteur de protéine, par exemple un inhibiteur de la trypsine du soja (STI) ou l'alpha-2-microglobuline.

11. Procédé selon la revendication 2, caractérisé en ce qu'on choisit la membrane (13) ou autre moyen de filtration et l'inhibiteur (7) en rapport l'un avec l'autre, de manière qu'une partie substantielle de l'inhibiteur (7) soit empêchée de passer à travers la membrane (13).

12. Procédé selon l'une quelconque des revendications précédentes, caractérisé en ce que les mesures de ladite substance sont combinées avec une mesure de la quantité totale de masse cellulaire sèche dans le milieu (2) qui fait l'objet du processus.

13. Procédé selon la revendication 3, caractérisé en ce qu'on choisit comme réacteur enzymatique (19) un réacteur dans lequel l'enzyme est attachée à un support solide, par exemple des perles de verre poreux.

14. Procédé selon l'une quelconque des revendications précédentes, caractérisé en ce qu'on choisit une sonde à double passage (4) comme instrument d'échantillonnage, dans lequel un passage (4b) est utilisé pour transporter l'inhibiteur vers un point situé au voisinage du point d'échantillonnage, tandis que l'autre passage (4a) est utilisé pour transporter l'échantillon liquide avec l'inhibiteur (7) vers le point d'analyse.

15. Procédé selon la revendication 14, caractérisé en ce qu'on choisit comme sonde à double passage (4) une sonde qui se compose de deux tubes concentriques, le tube interne (4a) se fermant à une courte distance de l'extrémité de la sonde à l'intérieur du tube externe (4b), le tube externe (4b) étant utilise de façon appropriée pour fournir l'inhibiteur.

**Patentansprüche**

1. Verfahren zur Kontrolle eines mikrobiologischen Verfahrens in einem Reaktor, in welchem eine Substanz verbraucht oder gebildet wird, wobei die Konzentration dieser Substanz kontinuierlich oder intermittierend als ein Maß des Fortschrittes oder Zustandes des Verfahrens gemessen wird und wobei eine flüssige Probe von dem Medium (2), welches Gegenstand des Verfahrens ist, zu einem Analysepunkt (18) in einem Abstand von dem tatsächlichen Platz des Verfahrens überführt wird, dadurch gekennzeichnet, daß die flüssige Probe mit einem Inhibitor (7) nahe dem Probenabnahmepunkt (4), vorzugsweise in dem Reaktor selbst vermischt wird, um so zu verhindern, daß das Verfahren während des Transportes von dem Probenabnahmepunkt (4) zu dem Analysepunkt (18) voranschreitet.

2. Verfahren nach Anspruch 1, dadurch gekennzeichnet, daß die flüssige Probe durch Dialyse oder andere Filtration in einen zellfreien Zustand gebracht wird.

3. Verfahren nach Anspruch 1 oder 2, dadurch gekennzeichnet, daß man die flüssige Probe durch einen Enzymreaktor (19) mit einem Enzym gehen läßt, welches die gemessene Substanz in ein leichter meßbares Produkt umwandelt oder welches ein leicht meßbares Co-Substrat, z.B. Sauerstoff, welcher mit Hilfe einer einfachen Sauerstoffelektrode gemessen wird, verbraucht.

4. Verfahren nach einem der vorausgehenden Ansprüche, dadurch gekennzeichnet, daß die flüssige Probe an dem Enzymreaktor (19) vorbei in gleichen Abständen direkt zu dem Analysepunkt (18) für eine Nulleinstellung der verwendeten analytischen Einrichtung geführt wird.

5. Verfahren nach einem der vorausgehenden Ansprüche, dadurch gekennzeichnet, daß die flüssige Probe in gleichen Abständen durch eine Kalibrierlösung (23) für die Kalibrierung der verwendeten analytischen Einrichtung versetzt wird.

6. Verfahren nach einem der vorausgehenden Ansprüche, dadurch gekennzeichnet, daß als der Inhibitor (7) ein solcher aus der Gruppe der Cyanide, Azide und Schwermetallionen, wie Kupfer-, Silber- oder Quecksilberionen, ausgewählt wird.

7. Verfahren nach Anspruch 6, das in einem biotechnischen Prozeß für die Messung der gebildeten oder verbrauchten Glukose angewendet wird, dadurch gekennzeichnet, daß als ein Inhibitor Kaliumcyanid (7) verwendet wird.

8. Verfahren nach einem der Ansprüche 1 bis 5, dadurch gekennzeichnet, daß als der Inhibitor (7) eine Verdünnungsflüssigkeit mit einem pH-Wert, der derart ist, daß das tatsächliche Verfahren unterbrochen wird, ausgewählt wird.

9. Verfahren nach einem der Ansprüche 1 bis 5, das auf den enzymatischen Abbau von Stärke mit Hilfe von Amylase angewendet wird, dadurch gekennzeichnet, daß als der Inhibitor (7) Amylaseinhibitor gewählt wird.

10. Verfahren nach einem der Ansprüche 1 bis 5, das auf die Proteinhydrolyse mit Hilfe von Enzymen des Proteasetyps angewendet wird, dadurch gekennzeichnet, daß als der Inhibitor (7) ein Proteininhibitor, wie z.B. Sojabohnentrypsininhibitor (STI) oder Alpha-2-mikroglobulin, gewählt wird.

11. Verfahren nach Anspruch 2, dadurch gekennzeichnet, daß die Membran (13) oder das andere Filtermedium und der Inhibitor (7) in Bezug aufeinander so ausgewählt werden, daß ein wesentlicher Teil des Inhibitors (7) daran gehindert wird, durch die Membran (13) zu gehen.

12. Verfahren nach einem der vorausgehenden Ansprüche, dadurch gekennzeichnet, daß die Messungen der Substanz mit einer Messung der Gesamtmenge an Trockenzellenmasse in dem Medium (2), welches Gegenstand des Verfahrens ist, kombiniert werden.

13. Verfahren nach Anspruch 3, dadurch gekennzeichnet, daß als der Enzymreaktor (19) ein solcher gewählt wird, bei dem das Enzym an einem festen Träger, z.B. porösen Glasperlen, befestigt ist.

14. Verfahren nach einem der vorausgehenden Ansprüche, dadurch gekennzeichnet, daß ein Doppel-Hohlraum-Katheter (4) als Probennahmeinstrument gewählt wird, in welchem ein Hohlraum (4b) zum Transportieren des Inhibitors zu einem Punkt nahe dem Probennahmepunkt verwendet wird, während der andere Hohlraum (4a) zum Transportieren der flüssigen Probe zusammen mit dem Inhibitor (7) zu dem Analysepunkt verwendet wird.

15. Verfahren nach Anspruch 14, dadurch gekennzeichnet, daß als der Doppel-Hohlraum-Katheter (4) ein solcher gewählt wird, der aus zwei konzentrischen Rohren besteht, wobei das Innenrohr (4a) in einem kurzen Abstand von der Spitze des Katheters im Inneren des Außenrohres (4b) endet und zweckmäßig das Außenrohr (4b) für die Zufuhr des Inhibitors verwendet wird.

Fig.1

EP 0 323 562 B1

Fig. 2

*Fig. 3*

*Fig. 4*